# EUROPEAN PATENT APPLICATION

(11) **EP 4 595 895 A1**
(43) Date of publication of application: **06.08.2025**
(21) Application number: 23886082.9
(22) Date of filing: 18.10.2023
(51) Int. Cl.: A61B 8/00, A61B 8/08

(54) **PORTABLE ULTRASONIC DEVICE FOR DETECTING TUMOR MASS IN BREAST THROUGH OPERATION CONTROL OF PLURALITY OF ULTRASONIC TRANSDUCERS**

(30) Priority: 31.10.2022 KR 20220142476; 31.10.2022 KR 20220142484; 31.10.2022 KR 20220142488; 31.10.2022 KR 20220142510; 18.09.2023 KR 20230123933
(71) Applicant: L'imagin Inc., Seoul 06687 (KR)
(72) Inventor: KIM, Kyoung Min, Seoul 06702 (KR)
(74) Representative: BCKIP Part mbB
(86) International application number: PCT/KR2023/016142
(87) International publication number: WO 2024/096385

(57) **Abstract**

According to embodiments of the present disclosure, a handheld ultrasound device configured to detect a breast mass, comprising: a transducer cover having a contact area to be in contact with a user's breast and located on one end of the handheld ultrasound device; a contact part whose outer surface is located on at least a portion of the contact area; an ultrasound transducer unit including N number (N is a natural number of 2 or more) of ultrasound transducers each configured to irradiate an ultrasound signal through the outer surface of the contact part and receive the reflected ultrasound signal; a controller configured to output a control signal to control the ultrasound transducer unit and generate ultrasound image information based on the received ultrasound signal; and a communication unit configured to transmit the ultrasound image information to an external device, wherein the controller turns on only one ultrasound transducer while turning off the others within one cycle T and generates ultrasound image information based on an ultrasound signal received from the turned-on ultrasound transducer.

## Description

### TECHNICAL FIELD

The present disclosure relates to a handheld ultrasound device for imaging breast tissue of a user and detecting a breast mass by using ultrasound.

### BACKGROUND

With recent advancements in medical equipment manufacturing technology, digital image processing technology has been applied to the clinical diagnosis field, which leads to significant developments in diagnostic radiology. In particular, ultrasound diagnosis is harmless to the human body because it avoids harmful radiation exposure compared to CT or X-ray medical equipment. Also, it provides cross-sectional imaging of the human body by using a non-invasive method and is portable and inexpensive.

In particular, ultrasound diagnosis can provide real-time images and thus enables the observation of organ movements in real-time. Such ultrasound diagnosis technology is widely used for breast cancer screening along with X-ray mammography.

However, if a posture (tilt, incident angle, contact pressure, and location) of an ultrasound scanner is not proper during ultrasound diagnosis for detecting a mass within the breast, it is impossible to obtain high-quality ultrasound images due to low resolution and severe noise. Further, when the ultrasound scanner operates, it is required to adjust the contact pressure and tilt for each affected area and it is virtually difficult for an untrained user to handle the ultrasound scanner. Therefore, it is impractical to perform a self-examination at home. Furthermore, only skilled experts can identify a suspicious mass, which may be cancer, in ultrasound images.

An ultrasound scanner designed for medical experts is equipped with a complex array of ultrasound elements which require precise control algorithms during operation. As a result, the prices of devices for detecting a breast mass increase, which results in reduction of accessibility for general users who seek to detect a breast mass.

Breast cancer has a high cure rate if detected early. As awareness of self-examination for breast cancer grows worldwide, various self-palpation methods have become widely known. However, since these methods do not utilize specialized medical equipment, there are many problems with measurement accuracy and systematic data management. Further, even when breast palpation is performed by a specialist in the field, its accuracy is less than 50%.

### DISCLOSURE OF THE INVENTION

### PROBLEMS TO BE SOLVED BY THE INVENTION

In view of the foregoing, the present disclosure is conceived to provide a handheld ultrasound device that allows a user who wants to perform self-examination for breast cancer to move an ultrasound scanner at a consistent speed according to the guidance provided by the ultrasound scanner and facilitates the generation of accurate ultrasound images of a breast mass.

Also, the present disclosure is conceived to provide a handheld ultrasound device that includes an ultrasound scanner equipped with a relatively small number of ultrasound transducers, and, thus, it is possible to reduce the production costs and unit price of the ultrasound scanner and also possible to enhance portability, reduce battery consumption, and improve user accessibility for breast cancer self-examination.

Further, the present disclosure is conceived to provide a handheld ultrasound device that transmits ultrasound images captured by an ultrasound scanner to a user's mobile device, allows the user to check the ultrasound images in real time without the need for separate display equipment, and thus enhances the convenience of breast cancer self-examination.

However, the problems to be solved by the present disclosure are not limited to the above-described problems. There may be other problems to be solved by the present disclosure.

### MEANS FOR SOLVING THE PROBLEMS

According to an aspect of the present disclosure, a handheld ultrasound device includes: a transducer cover having a contact area to be in contact with a user's breast and located on one end of the handheld ultrasound device; a contact part whose outer surface is located on at least a portion of the contact area; an ultrasound transducer unit including N number (N is a natural number of 1 or more) of ultrasound transducers each configured to irradiate an ultrasound signal through the outer surface of the contact part and receive the reflected ultrasound signal; a movement speed sensing unit configured to sense a movement speed of the contact area in contact with the breast and generate movement speed information; a controller configured to output a control signal to control the ultrasound transducer unit and generate ultrasound images based on the received ultrasound signal; and a communication unit configured to transmit the ultrasound images to an external device.

According to another aspect of the present disclosure, a handheld ultrasound device includes: a transducer cover having a contact area to be in contact with a user's breast and located on one end of the handheld ultrasound device; a contact part whose outer surface is located on at least a portion of the contact area; an ultrasound transducer unit including N number (N is a natural number of 2 or more) of ultrasound transducers each configured to irradiate an ultrasound signal through the outer surface of the contact part and receive the reflected ultrasound signal; a controller configured to output a control signal to control the ultrasound transducer unit and generate ultrasound images based on the received ultrasound signal; and a communication unit configured to transmit the ultrasound images to an external device, and the controller turns on only one ultrasound transducer while turning off the others within one cycle T and generates an ultrasound image based on an ultrasound signal received from the turned-on ultrasound transducer.

The above-described aspects are provided by way of illustration only and should not be construed as liming the present disclosure. Besides the above-described embodiments, there may be additional embodiments described in the accompanying drawings and the detailed description.

### EFFECTS OF THE INVENTION

According to any one of the above-described means for solving the problems of the present disclosure, it is possible to allow a user who wants to perform self-examination for breast cancer to move an ultrasound scanner at a consistent speed according to the guidance provided by the ultrasound scanner and facilitate the generation of accurate ultrasound images.

Also, the ultrasound scanner is equipped with a relatively small number of ultrasound transducers, and, thus, it is possible to reduce the production costs and unit price of the ultrasound scanner and also possible to improve user accessibility for breast mass detection.

Further, it is possible to transmit ultrasound images captured by the ultrasound scanner to the user's mobile device, allow the user to check the ultrasound images in real time without the need for separate display equipment, and thus enhance the convenience of breast mass detection.

### BRIEF DESCRIPTION OF THE DRAWINGS

FIG. 1 is a configuration diagram of an ultrasound breast cancer diagnosis system including a handheld ultrasound device according to an embodiment of the present disclosure.
FIG. 2 is a diagram for explaining a configuration of the handheld ultrasound device according to an embodiment of the present disclosure.
FIG. 3 and FIG. 4 are diagrams for explaining a scan operation of the handheld ultrasound device according to an embodiment of the present disclosure.
FIG. 5 and FIG. 6 are diagrams for explaining a control signal of the handheld ultrasound device according to an embodiment of the present disclosure.
FIG. 7A, FIG. 7B, FIG. 8A and FIG. 8B are other diagrams for explaining an ultrasound transducer of the handheld ultrasound device according to an embodiment of the present disclosure.
FIG. 9 is another diagram for explaining an interface screen of a mobile device on which ultrasound images generated by the handheld ultrasound device are displayed according to an embodiment of the present disclosure.
FIG. 10 to FIG. 14 are diagrams for explaining an example where axial movements are performed in the handheld ultrasound device according to an embodiment of the present disclosure.

### BEST MODE FOR CARRYING OUT THE INVENTION

Hereafter, example embodiments will be described in detail with reference to the accompanying drawings so that the present disclosure may be readily implemented by those skilled in the art. However, it is to be noted that the present disclosure is not limited to the example embodiments but can be embodied in various other ways. In the drawings, parts irrelevant to the description are omitted for the simplicity of explanation, and like reference numerals denote like parts through the whole document.

Throughout this document, the term "connected to" may be used to designate a connection or coupling of one element to another element and includes both an element being "directly connected" another element and an element being "electronically connected" to another element via another element. Further, it is to be understood that the terms "comprises," "includes," "comprising," and/or "including" means that one or more other components, steps, operations, and/or elements are not excluded from the described and recited systems, devices, apparatuses, and methods unless context dictates otherwise; and is not intended to preclude the possibility that one or more other components, steps, operations, parts, or combinations thereof may exist or may be added.

Throughout this document, the term "unit" may refer to a unit implemented by hardware, software, and/or a combination thereof. As examples only, one unit may be implemented by two or more pieces of hardware or two or more units may be implemented by one piece of hardware.

Throughout this document, a part of an operation or function described as being carried out by a terminal or device may be implemented or executed by a device connected to the terminal or device. Likewise, a part of an operation or function described as being implemented or executed by a device may be so implemented or executed by a terminal or device connected to the device.

Hereinafter, an embodiment of the present disclosure will be described in detail with reference to the accompanying drawings.

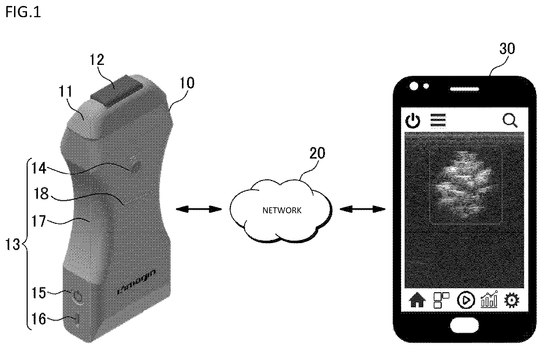

FIG. 1 is a configuration diagram of an ultrasound breast mass detection system including a handheld ultrasound device according to an embodiment of the present disclosure.

Referring to FIG. 1, the ultrasound breast mass detection system may include a handheld ultrasound device 10, a wireless network 20, and an external device 30.

Also, a transducer cover 11, a contact part 12, and a body part 13 may be provided on an outer surface of the handheld ultrasound device 10. The body part 13 may include a toggle switch 14, a power switch 15, a charging port 16, a handle 17, and an LED 18.

The handheld ultrasound device 10 may be connected to the external device 30 through a wired network or the wireless network 20. For example, the handheld ultrasound device 10 may be connected to the external device 30 simultaneously or sequentially through the network 20. Further, the term "network" refers to a connection structure that enables information exchange between nodes such as devices, servers, etc. and includes LAN (Local Area Network), WAN (Wide Area Network), Internet (WWW: World Wide Web), a wired or wireless data communication network, a telecommunication network, a wired or wireless television network, and the like. Examples of the wireless data communication network may include 3G, 4G, 5G, 3GPP (3rd Generation Partnership Project), LTE (Long Term Evolution), WIMAX (World Interoperability for Microwave Access), Wi-Fi, Bluetooth communication, infrared communication, ultrasonic communication, VLC (Visible Light Communication), LiFi, and the like, but may not be limited thereto.

The transducer cover 11 of the handheld ultrasound device 10 has a contact area to be in direct contact with a user's breast while the handheld ultrasound device 10 is being used. To this end, the transducer cover 11 may be located on one end of the handheld ultrasound device 10.

The contact part 12 includes an ultrasound transducer unit configured to irradiate an ultrasound signal and generate ultrasound images, and has an outer surface in an area including at least a portion of the contact area of the transducer cover 11. Also, the contact part 12 can be in direct contact with the user's skin during a scan of the handheld ultrasound device 10.

That is, the contact part 12 can cover one or more ultrasound transducers located therein, and may be formed of a material that provides appropriate acoustic impedance matching to an anatomical region or ensures proper focusing of ultrasound energy when the ultrasound energy is projected onto the anatomical region.

The body part 13 refers to the components of the handheld ultrasound device 10 excluding the transducer cover 11. The body part 13 may include the toggle switch 14, the power switch 15, the charging port 16, the handle 17, and the LED 18.

The body part 13 may include the toggle switch 14 configured to receive input from the user, the power switch 15 configured to turn on and off the handheld ultrasound device 10, the charging port 16 to be connected to a cable for charging a battery module inside the handheld ultrasound device 10, and the handle 17 for the user to grip.

The handle 17 allows the user to move the handheld ultrasound device 10 over the area of interest (e.g., the breast) by hand. To facilitate gripping, the handle 17 may have a concave-shaped contour.

By gripping the handle 17 of the handheld ultrasound device 10, the user can activate one or more ultrasound transducers located inside the transducer cover 11 and transmit ultrasound signals to the area of interest (e.g., a specific organ, joint, blood vessel, etc.) of a patient. For example, the transducer cover 11 may be located on the user's breast.

The LED 18 indicates a power status of the handheld ultrasound device 10 through an externally exposed LED. Also, the LED 18 may blink or output a specific color corresponding to movement speed information, which will be described later.

When the user's area of interest is scanned, the ultrasound transducer inside the contact part 12 may initiate an ultrasound scan of the selected anatomical region while in contact with the user's skin. In another embodiment, the scan of the ultrasound transducer may be initiated manually or automatically via a trigger operation. The trigger operation may be started manually or automatically by manipulating the toggle switch 14 attached to the outer surface of the handheld ultrasound device 10 or an interface inside the external device 30 wirelessly connected to the handheld ultrasound device 10.

Although not shown in FIG. 1, a display part may be provided in a portion of the body part 13 to display, in text or an image, information generated while the user's breast is being scanned. Also, a plurality of holes may be formed in a portion of the body part 13 so as to be adjacent to a speaker unit configured to provide voice guidance to the user or output sounds corresponding to movement speed control information while the user's breast is being scanned.

The external device 30 is connected to the handheld ultrasound device 10 through the network 20 for data transmission and reception. The external device 30 may be equipped with a display capable of receiving and displaying various types of information generated by the handheld ultrasound device 10 and provided through the network 20. Further, the external device 30 may transmit control signals to the handheld ultrasound device 10 through the network 20 under the control of the user. The external device 30 includes a mobile smartphone, a personal computer, or a medical device accessible to the network 20 and equipped with a display.

As another example, the external device 30 may include an artificial intelligence (AI) cloud server. The user can access the Al cloud server included in the external device 30 by using a separate end device, download data uploaded from the handheld ultrasound device 10 through the Al cloud server, or upload other data to the Al cloud server. The Al cloud server may provide the handheld ultrasound device 10 with commands based on the data uploaded by the user's end device or stored data.

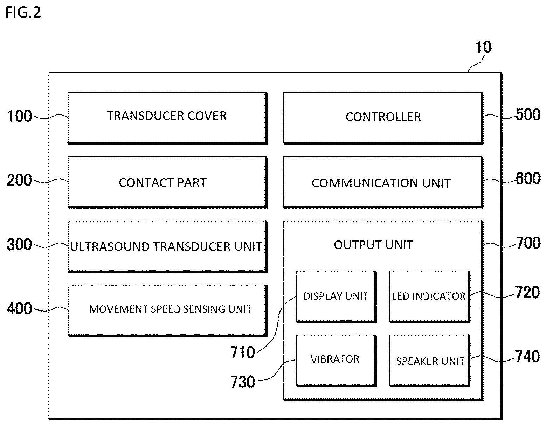

FIG. 2 is a diagram for explaining a configuration of the handheld ultrasound device according to an embodiment of the present disclosure.

Referring to FIG. 2, the handheld ultrasound device 10 may include a transducer cover 100, a contact part 200, a ultrasound transducer unit 300, a movement speed sensing unit 400, a controller 500, a communication unit 600, and an output unit 700.

As illustrated in FIG. 1, the transducer cover 100 and the contact part 200 may be located on an outer surface of the handheld ultrasound device 10. More specifically, the transducer cover 100 may have a contact area where the handheld ultrasound device 10 is to be in contact with the user's breast skin which is an examination target body part. Herein, the body part refers to the components of the handheld ultrasound device 10 excluding the transducer cover 100.

The transducer cover 100 may be provided on one end of the handheld ultrasound device 10, and the contact part 200 may be provided on at least a portion of the contact area. For example, the contact part 200 may be located at the center of the contact area of the transducer cover 100. The contact part 200 can cover the ultrasound transducer unit 300 located therein. The contact part 200 may have a protruding shape compared to a surface corresponding to the contact area of the transducer cover 100. Accordingly, when the user views the external surface of the handheld ultrasound device 10, he/she can more easily identify a location of the contact part 200 corresponding to the actual scan area and can also perform a more precise ultrasound scan on the breast.

The ultrasound transducer unit 300 irradiates ultrasound signals through the outer surface of the contact part 200 and may include at least two ultrasound transducers arranged horizontally within the transducer cover 100.

The ultrasound transducer unit 300 may accommodate one or more ultrasound transducers. Each ultrasound transducer may generate ultrasound energy at a specific frequency and/or pulse repetition rate, receive reflected ultrasound energy (e.g., ultrasound echoes), and convert the reflected ultrasound energy into electrical signals.

For example, the ultrasound transducer unit 300 may be configured to transmit ultrasound signals within a predetermined frequency range. The ultrasound transducer unit 300 may transmit pulse signals to the respective ultrasound transducers based on received pulse control information. Also, the ultrasound transducer unit 300 may accommodate a plurality of ultrasound transducers arranged in one or more rows.

The ultrasound transducer unit 300 may also include a transceiver circuitry including a transmitter and a receiver respectively configured to transmit and receive ultrasound signals.

The movement speed sensing unit 400 may sense a movement speed of the contact area of the transducer cover 100 in contact with the breast and generate movement speed information. For example, the movement speed sensing unit 400 includes at least one of a speed calculator that calculates a speed of the ultrasound transducer inside the handheld ultrasound device based on a change in received ultrasound signal, an image sensor, an acceleration sensor, or an optical sensor.

If the movement speed sensing unit 400 functions as a speed calculator, it can measure a movement speed based on a change in received ultrasound signal. Herein, the ultrasound signals may be generated by the ultrasound transducer unit 300. The ultrasound signals may have one-dimensional values measured at predetermined time intervals by the ultrasound transducer unit 300.

The process of calculating a speed based on ultrasound signals by the speed calculator may be defined as follows in Equation 1.

<Equation 1>

Herein, Δ may represent a change in position, and yk may correspond to a value sampled according to the sampling time order of an analog-to-digital converter from a signal measured at a first position of the ultrasound transducer unit 300 by the speed calculator (where k is a natural number between 1 and n, and n is the number of ultrasound transducers). Also, y'k may correspond to a value sampled according to the sampling time order of the analog-to-digital converter from a signal measured at a second position of the ultrasound transducer unit 300 by the speed calculator (where k is a natural number between 1 and n, and n is the number of ultrasound transducers). Also, a time interval between yk and yk-1 may be defined as equal to a time interval between y'k and y'k-1.

If the ultrasound transducer does not move, Δ is theoretically derived as 0 or may be a value close to 0 due to a change in noise or micro signal.

If the ultrasound transducer moves, Δ increases. As a change in position of the ultrasound transducer increases, Δ has a greater value. The speed calculator can measure a speed of the ultrasound transducer unit based on the calculated Δ.

If the movement speed sensing unit 400 includes an image sensor, the image sensor may capture images of the contact area of the transducer cover 100 or its surrounding area with a camera exposed on the outer surface of the handheld ultrasound device 10. The movement speed sensing unit 400 may calculate a speed of the handheld ultrasound device 10 based on a change in images generated at predetermined time intervals by the image sensor. For example, the speed calculator may calculate a speed of the handheld ultrasound device through an object tracking algorithm, a feature point detection algorithm, or an optical flow estimation algorithm.

The object tracking algorithm is an algorithm that tracks a position of a specific point in consecutively captured images by image processing technology. The speed calculator may continuously track a specific object or feature point in the images through the object tracking algorithm and calculate movement vectors based on the tracked information.

The feature point detection algorithm detects feature points (which are unchanged features) in an image and matches the feature points with those in another image to determine a change in position. Examples of the feature point detection algorithm may include Scale-Invariant Feature Transform (SIFT) and Speeded-Up Robust Features (SURF). The speed calculator may extract feature points from an image and match them with corresponding feature points in another image to estimate a change in position.

The optical flow estimation algorithm analyzes a pixel movement pattern between adjacent frames in an image to estimate movement vectors. Optical flow provides information on how pixels move. The speed calculator may use the optical flow estimation algorithm to estimate movement vectors and calculate a movement speed based on the movement distance and time interval.

If the movement speed sensing unit 400 functions as an acceleration sensor, the acceleration sensor can detect a movement speed of the handheld ultrasound device 10. For example, if the acceleration sensor is an acceleration integrated circuit (IC) based on Micro Electro Mechanical Systems (MEMS) technology, it may be a capacitive MEMS accelerometer or a piezoresistive MEMS accelerometer and may measure acceleration in response to a movement of the handheld ultrasound device 10. The movement speed sensing unit 400 may calculate a movement distance of the handheld ultrasound device 10 over a predetermined time based on the acceleration detected by the acceleration sensor and generate movement speed information including a current speed.

If the movement speed sensing unit 400 functions as an optical sensor, the optical sensor may be provided inside the handheld ultrasound device 10. When the user operates the handheld ultrasound device 10, it can perform sensing while facing the breast skin. By analyzing the generated information in a time-series manner, a change of images over time can be calculated. More specifically, the optical sensor may be an optical sensor or an optical flow sensor using LEDs. The optical sensor may illuminate the skin with an LED (or laser) inside the handheld ultrasound device 10 and detect a change in skin pattern as the handheld ultrasound device 10 moves. Based on this pattern change, the movement speed sensing unit 400 may track a movement of the handheld ultrasound device 10 and generate movement speed information. The optical flow sensor may detect a change in position by tracking a pixel movement between consecutive image frames. Therefore, the movement speed sensing unit 400 may calculate a movement direction and a movement speed of the handheld ultrasound device 10 to generate movement speed information. Also, the movement speed sensing unit 400 may apply at least one of the object tracking algorithm, feature point detection algorithm or optical flow estimation algorithm to a plurality of images generated by the above-described method to calculate a speed of the handheld breast mass detection device 10.

The movement speed information generated by the movement speed sensing unit 400 may be provided to the output unit 700, and the output unit 700 may process the movement speed information into another type of information and output it to the user holding the handheld breast mass detection device 10. Alternatively, the movement speed information generated by the movement speed sensing unit 400 may be provided to the communication unit 600, and the communication unit 600 may transmit the movement speed information to the external device 30.

The controller 500 may output a control signal to control the ultrasound transducer unit 300 and generate ultrasound images based on the ultrasound signals received from the ultrasound transducer unit 300. Further, the controller 500 may turn on only one ultrasound transducer among the plurality of ultrasound transducers included in the ultrasound transducer unit 300 while turning off the others within one cycle T and generate an ultrasound image based on an ultrasound signal received from the turned-on ultrasound transducer.

Furthermore, in a first cycle, the controller 500 may turn on only a first ultrasound transducer, and in a second cycle, it may turn on only a second ultrasound transducer. Herein, the on-periods of the first and second ultrasound transducers may not overlap.

The cycle T may be determined based on a value obtained by dividing a measurement depth of the ultrasound transducer by a propagation speed of ultrasound in the breast.

Signal phase differences among N number of ultrasound transducers may be determined independently of the number N and shape of the ultrasound transducers, and a spacing between the ultrasound transducers.

The ultrasound transducer unit 300 may sequentially and individually transmit ultrasound signals from the N number of ultrasound transducers to the controller 500. The controller 500 may receive the ultrasound signals sequentially and individually from the N number of ultrasound transducers and generate ultrasound images sequentially and individually.

The control signal may be independent of a cross-sectional size of each of the N number of ultrasound transducers and a spacing between the N number of ultrasound transducers.

Further, the controller 500 may correct the generated ultrasound images based on the movement speed information generated by the movement speed sensing unit 400.

A more detailed description of the above-described operations of the controller 500 will be provided later.

The communication unit 600 may transmit, to the external device 30, the ultrasound images generated by the controller 500. The communication unit 600 may also receive data or commands from the external device 30. The communication unit 600 may transmit, to the external device 30, the movement speed information generated by the movement speed sensing unit 400. The external device 30 may output the movement speed information to the user through its display. Herein, the movement speed information of the handheld ultrasound device 10 may be processed into text or an image corresponding to a movement speed status and displayed on the external device 30.

The output unit 700 may display information generated by the handheld breast mass detection device 10 to the outside of the handheld ultrasound device 10. More specifically, the output unit 700 may output information corresponding to the movement speed information.

The output unit 700 may include at least one of a display unit 710 configured to output an image or text corresponding to the movement speed information, an LED indicator 720 configured to blink or output a color in response to the movement speed information, a vibrator 730 configured to vibrate according to the movement speed information, and a speaker unit 740 configured to output a sound corresponding to the movement speed information.

The display unit 710 may display the measured movement speed of the handheld ultrasound device 10 in text or an image corresponding to a movement speed status. Therefore, the user can check in real time whether a scan is appropriately performed while moving the handheld breast mass detection device 10 at a proper speed. Also, the user can obtain an optimal scan result by controlling a scan speed in response to the sound.

The LED indicator 720 may emit light after adjusting the repetition rate, brightness, or color of light to be emitted depending on whether or not the movement speed is within a predetermined normal range. More specifically, when the movement speed is within the predetermined range, the LED indicator 720 may repeatedly emit light of at least one color. If the movement speed is determined to be out of the predetermined range, indicating that the movement speed is too slow or too fast, the LED indicator 720 may change at least one of the brightness, color, and repetition rate of light to be emitted. Therefore, the user can check in real time whether a scan is appropriately performed while moving the handheld ultrasound device 10 at a proper speed. Also, the user can obtain an optimal scan result by controlling a scan speed in response to the sound.

The vibrator 730 may output a vibration after adjusting the interval, intensity, or pattern of vibration to be output depending on whether or not the movement speed is within a predetermined normal range. More specifically, when the movement speed is within the predetermined range, the vibrator 730 may output vibrations, which are weak enough not to interfere with scanning, at regular intervals and lengths. If the movement speed is determined to be out of the predetermined range, indicating that the movement speed is too slow or too fast, the vibrator 730 may change the interval, intensity, or pattern of vibrations to be output. Therefore, the user can check in real time whether a scan is appropriately performed while moving the handheld ultrasound device 10 at a proper speed. Also, the user can obtain an optimal scan result by controlling a scan speed in response to the sound.

The speaker unit 740 may output a sound after adjusting at least one of the interval, volume, and type of sound to be output depending on whether or not the movement speed is within a predetermined normal range. More specifically, when the movement speed is within the predetermined range, the speaker unit 740 may repeatedly output a short sound or a simple rhythmic sound. If the movement speed is determined to be out of the predetermined range, indicating that the movement speed is too slow or too fast, the speaker unit 740 may change at least one of the interval, volume, and type of sound to be output. Also, the speaker unit 740 may provide voice guidance to output information corresponding to the movement speed. Therefore, the user can check in real time whether a scan is appropriately performed while moving the handheld ultrasound device 10 at a proper speed. Also, the user can obtain an optimal scan result by controlling a scan speed in response to the sound.

The movement speed information includes information about the movement speed of the handheld ultrasound device 10, and the handheld ultrasound device 10 can determine whether or not its movement speed is within a predetermined range. For example, the movement speed status may be determined based on whether the handheld ultrasound device 10 moves at a speed lower than a predetermined reference movement speed range, within the reference movement speed range, or higher than the reference movement speed range.

Further, the handheld ultrasound device 10 can notify the user of a current measured movement speed status through the LED indicator 14. For example, when the LED indicator 14 is lit in red, it indicates that the current movement speed is too fast, whereas when the LED indicator 14 is lit in yellow, it indicates that the current movement speed is too slow. Further, the handheld ultrasound device 10 may notify the user of the movement speed status by making the LED indicator 14 blink at specific intervals. For example, when the LED indicator 14 blinks rapidly, it indicates that the current movement speed is fast, whereas when the LED indicator 14 blinks slowly, it indicates that the current movement speed is slow.

Hereafter, operations of the respective components included in the handheld ultrasound device 10 will be described in more detail with reference to the accompanying drawings and the following description.

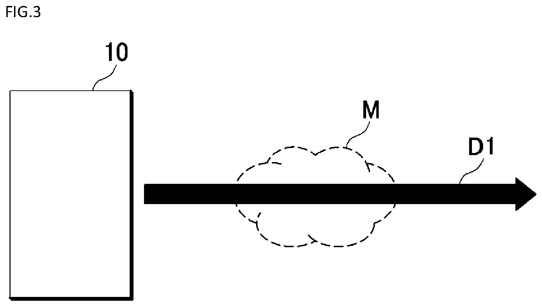

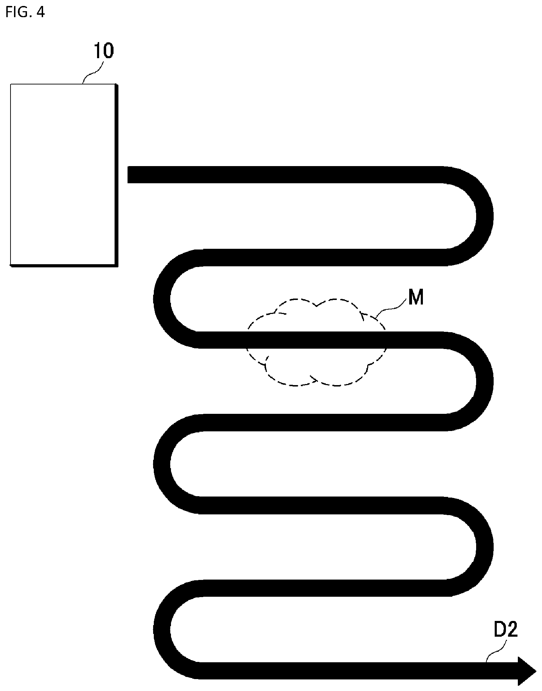

FIG. 3 and FIG. 4 are diagrams for explaining a method of using the handheld ultrasound device according to an embodiment of the present disclosure.

To provide an acoustic impedance match when the contact part 200 is placed on the breast skin, the user may apply an acoustic gel to the breast skin. Then, the user may select to perform an ultrasound scan on the breast by pressing the toggle switch of the handheld ultrasound device 10, by entering a scan start command on the external device 30, by speaking a voice command, or using another type of scan activation technique. In response, the handheld ultrasound device 10 may transmit ultrasound signals into the breast and receive reflected ultrasound signals. The reflected ultrasound signals may be processed into images displayed on the external device 30.

Referring to FIG. 3, the handheld ultrasound device 10 moves in a certain direction D1 over the user's breast skin. More specifically, to move the handheld ultrasound device 10, the user can bring the contact part 12 into contact with the breast skin while holding the handheld ultrasound device 10. Then, the user can turn on the handheld ultrasound device 10 and move his/her arm or hand while keeping the contact part 12 in contact with the breast to move the handheld ultrasound device 10. During the movement, the handheld ultrasound device 10 irradiates ultrasound signals into the user's breast through the contact part 12 and receives reflected ultrasound signals based on the location and shape of internal breast tissues to generate ultrasound images. If a mass M, which can be a potential cause of breast cancer, is located within a movement path of the handheld ultrasound device 10, the handheld ultrasound device 10 may generate ultrasound images including the mass M.

Referring to FIG. 4, it can be seen that the handheld ultrasound device 10 changes its movement direction D2 on the user's skin and performs an ultrasound scan over a wide area compared to the example shown in FIG. 3. To scan a wider area, the user can scan the breast tissues within the movement path by moving the handheld ultrasound device 10 from one point to another, changing the direction of the handheld ultrasound device 10 and moving the handheld ultrasound device 10 between points in another area to locate the mass M.

As described below, ultrasound signals are transmitted and received by a single ultrasound transducer at predetermined time intervals. Thus, the user needs to move the handheld ultrasound device 10 at a consistent speed to obtain accurate ultrasound images for each scan area.

To this end, when the ultrasound transducer unit 300 irradiates ultrasound signals, the movement speed sensing unit 400 senses a movement speed of the handheld ultrasound device 10 and generates movement speed information accordingly.

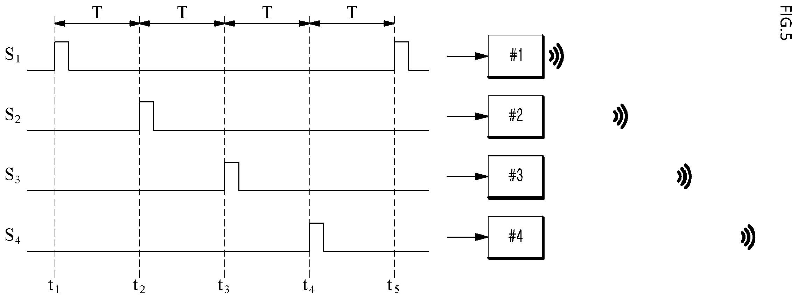

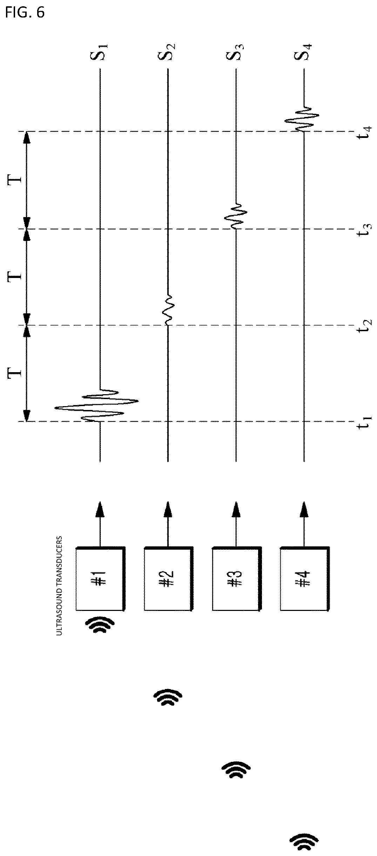

FIG. 5 and FIG. 6 are diagrams for explaining a process in which a controller of the handheld ultrasound device transmits a control signal to control an operation of an ultrasound transducer according to an embodiment of the present disclosure.

The controller 500 may transmit a control signal including pulse signals to control each of ultrasound transducers S1 to Sk of the ultrasound transducer unit 300 to irradiate ultrasound signals according to a predetermined pulse pattern. FIG. 5 is a diagram illustrating pulse signals included in a control signal and a plurality of ultrasound transducers irradiating ultrasound signals in response to the pulse signals. FIG. 5 illustrates the pulse signals for the plurality of ultrasound transducers S1 to Sk included in pulse control information and operation time points for the ultrasound transducers S1 to Sk defined by the pulse signals.

The control signal may include the pulse signals provided for each ultrasound transducer to control the operation time points for the plurality of ultrasound transducers. The control signal may include information about an operating sequence of the ultrasound transducers in order for the ultrasound transducers to operate sequentially.

The operating sequence of the ultrasound transducers follows a predetermined sequence of the ultrasound transducers S1, S2, S3 ... Sk. The sequence of the ultrasound transducers S1 to Sk may start from, for example, one end of the ultrasound transducer unit 400. Herein, k represents the number of ultrasound transducers included in the ultrasound transducer unit 400. In FIG. 5 and FIG. 6, k is illustrated as having a value of 4.

A first ultrasound transducer S1 in the ultrasound transducer unit 400 may operate at a first time point t1 in response to a pulse signal. A second ultrasound transducer S2 may operate at a second time point t2 in response to a pulse signal, and a third ultrasound transducer S3 may operate at a third time point t3 in response to a pulse signal. Likewise, a k-th ultrasound transducer S4 may operate at a fourth time point t4 in response to a pulse signal. Then, the first ultrasound transducer S1 may operate again at a fifth time point t5.

A time interval between consecutive time points can be defined as the cycle T. A time interval between the first time point t1 and the second time point t2 may differ by T, and a time interval between the second time point t2 and the third time point t3 may also differ by T. Therefore, a k-th time point tk at which the k-th ultrasound transducer operates may differ from the first time point t1 by (k-1)*T. That is, signal phase differences among the ultrasound transducer S1 to S4 can be fixed to the cycle T, regardless of the number, shape, or spacing of the ultrasound transducers. The control signal transmitted to the k number of ultrasound transducers allows the ultrasound transducers to sequentially and individually transmit ultrasound signals.

Herein, the cycle T may correspond to the time required for a single ultrasound transducer to complete its operation. For example, the operation of each ultrasound transducer may include a process of irradiating ultrasound signals and receiving reflected ultrasound signals from the breast tissues. The controller 500 may transmit pulse signals through the control signal to ensure that the ultrasound transducers operate sequentially at intervals of the cycle T. Therefore, only one ultrasound transducer can operate during a single cycle T. That is, different ultrasound transducers are controlled to irradiate ultrasound signals at different times. Operation periods of the ultrasound transducers may not overlap. Consequently, after a one-round operation of a single ultrasound transducer is completed, it may take a time of k*T for the next operation to be performed. Referring to FIG. 5, after the first ultrasound transducer S1 operates at the first time point t1, it operates again at the fifth time point t5, which differs from the first time point by 4 T.

Herein, the cycle T corresponds to an operation period of each ultrasound transducer. Therefore, it can be determined based on a value obtained by dividing a breast depth to be measured by the handheld ultrasound device 10 by a propagation speed of ultrasound in the breast.

**The** controller 500 may transmit a control signal to turn on only the first ultrasound transducer S1 during a first cycle (from t1 to t2) and then turn on only the second ultrasound transducer S2 during a second cycle (from t2 to t3). In this case, the on-periods of the first ultrasound transducer S1 and the second ultrasound transducer S2 do not overlap. Similarly, while the third ultrasound transducer S3 is turned on during a third period (from t3 to t4), the second ultrasound transducer S2 is turned off. That is, the control signal may be provided to ensure that when a new ultrasound transducer is turned on, the previous ultrasound transducer is turned off.

In a professional use ultrasound device equipped with tens to hundreds of ultrasound transducers, a control signal for a plurality of ultrasound transducers is transmitted during a single cycle T, which is determined based on the measurement depth. Consequently, the signals received from the ultrasound transducers have phase differences, and time delays need to be set based on a cross-sectional size of each ultrasound transducer, a spacing between the ultrasound transducers, and a desired beam shape. According to the conventional technology, a plurality of ultrasound signals generated by the professional use ultrasound device is combined into a single signal to generate an ultrasound image. According to the conventional technology, a more complex process is required to generate a control signal in consideration of all the cross-sectional size of each ultrasound transducer, the spacing between the ultrasound transducers, and the desired beam shape, which causes an increase in design complexity of the ultrasound system and ultimately raises the overall production costs of the ultrasound device.

In contrast, in a handheld ultrasound device equipped with 10 or fewer ultrasound transducers according to an embodiment of the present disclosure, only one ultrasound transducer is turned on during a single cycle T. Therefore, signal phase differences among the ultrasound transducer can be fixed to the predetermined cycle T, regardless of the number, shape, or spacing of the ultrasound transducers. Also, ultrasound signals are transmitted sequentially and individually and used to generate separate ultrasound images. For example, K number of ultrasound signals may be received sequentially and individually at time points t1, t2, ... tk to generate K number of images. Herein, the cycle T is determined as being equal to tk - t(k-1). Therefore, it is possible to decrease the design complexity of the ultrasound system and thus reduce the overall production costs of the ultrasound device. Referring to FIG. 6, the ultrasound signals are transmitted according to the pulse signals transmitted to the ultrasound transducers S1 to S4 in the ultrasound transducer unit 300 via the control signal generated by the controller 500 and the reflected ultrasound signals are received.

Each of the ultrasound transducers S1 to S4 can complete the transmission of ultrasound signals and the reception of reflected ultrasound signals during the cycle T. FIG. 6 illustrates time points at which the respective ultrasound transducers S1 to S4 receive ultrasound signals. It can be seen from FIG. 6 that during each cycle T, only one ultrasound transducer receives a reflected ultrasound signal. The received ultrasound signals may be transmitted to the controller 500, and the controller 500 may generate ultrasound images based on the received ultrasound signals. During the cycle T, a single ultrasound transducer may irradiate an ultrasound image for scanning and receive a reflected ultrasound signal. When k number of ultrasound transducers receive reflected ultrasound signals and transmit them to the controller 500, it may take a time of k*T.

**The** controller 500 may receive the ultrasound signals sequentially and individually from N number of ultrasound transducers in the ultrasound transducer unit 300 and generate ultrasound images sequentially and individually. That is, each of the ultrasound transducers can individually transmit the reflected ultrasound signals to the controller 500. Such individual transmission may be performed sequentially according to the sequence in which the ultrasound transducers are turned on.

The control signal provided by the controller 500 may be independent of a cross-sectional size of each of ultrasound transducers included in the ultrasound transducer unit 300 and a spacing between the ultrasound transducers. The control signal may be provided independently to each ultrasound transducer. The independently provided control signal may include pulse signals for controlling the on and off timings of the ultrasound transducers. The control signal can be provided independently of factors, such as variations in cross-sectional size of each ultrasound transducer or spacing between the ultrasound transducers.

More specifically, in the professional use ultrasound device equipped with tens to hundreds of ultrasound transducers, each ultrasound transducer has a rectangular cross-section, and the characteristics of control signals for adjacent ultrasound transducers vary depending on the size, spacing, and shape of each cross-section. As a result, the configuration of the ultrasound system becomes complex and the maintenance costs of the system increases.

In contrast, in the handheld ultrasound device 10 equipped with 10 or fewer ultrasound transducers according to an embodiment of the present disclosure, the controller 500 maintains only one ultrasound transducer in a turned-on state during a single cycle and thus can provide independent and consistent control signals regardless of the cross-sectional shape, size, or spacing of the ultrasound transducers. Accordingly, there is no need to reflect all the characteristics of the ultrasound transducers, and it is possible to more easily and simply design control signals and also reduce the maintenance costs of the ultrasound device.

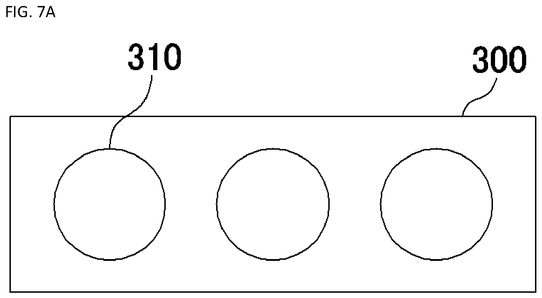

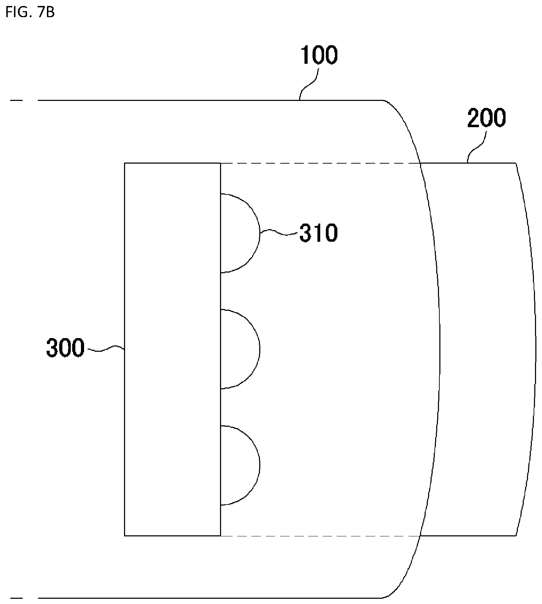

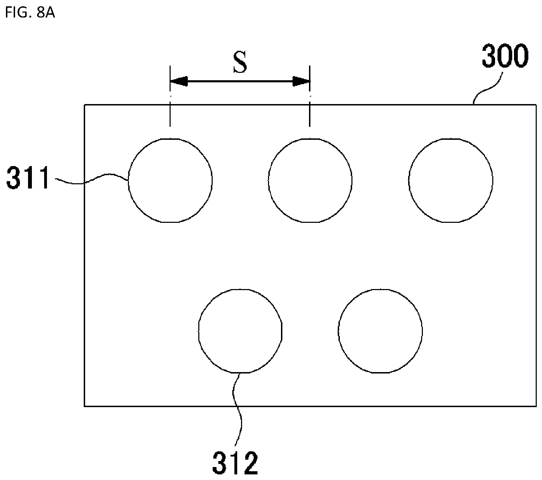

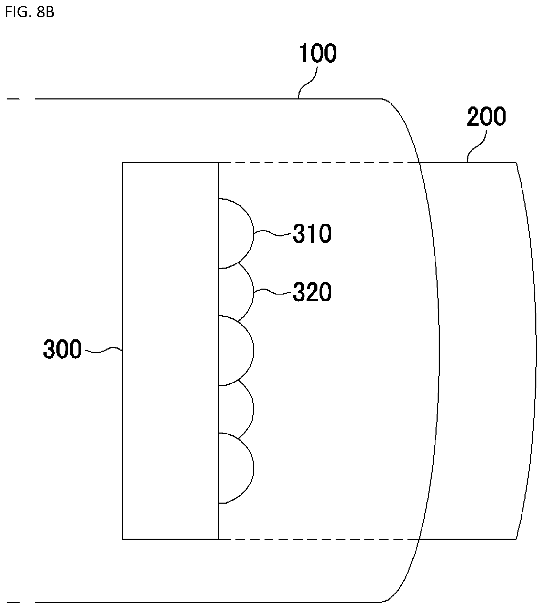

FIG. 7A, FIG. 7B, FIG. 8A and FIG. 8B are other diagrams for explaining an ultrasound transducer of the handheld ultrasound device according to an embodiment of the present disclosure.

**A** plurality of ultrasound transducers 310 included in the ultrasound transducer unit 300 may be arranged in a single row as shown in FIG. 7A. Further, as shown in FIG. 7B, the plurality of ultrasound transducers 310 in the ultrasound transducer unit 300 may be arranged in a direction toward a contact cover 200 from the ultrasound transducer unit 300 and may also be arranged in a direction parallel to the transducer cover 100. During a scan process of the handheld ultrasound device 10, the ultrasound transducers 310 can operate sequentially to irradiate ultrasound signals. In this case, the ultrasound transducers from one end to the other end of the ultrasound transducer unit 300 can operate sequentially. Although FIG. 7A and FIG. 7B illustrate an example where the ultrasound transducer unit 300 includes three ultrasound transducers 310, the number of ultrasound transducers may be adjusted between 1 and 10 depending on the design.

FIG. 7A and FIG. 8A illustrate front views of the ultrasound transducer unit 300. Herein, ultrasound transducers 310, 311 and 312 included in the ultrasound transducer unit 300 may each have a circular cross-section. In another embodiment, unlike the example shown in FIG. 7A and FIG. 8A, the ultrasound transducers 310, 311 and 312 may each have a rectangular cross-section.

**In** embodiments of the present disclosure, the number of ultrasound transducers included in the ultrasound transducer unit 300 can be designed to be up to 10.

If the number of ultrasound transducers ranges from tens to hundreds, the production costs of the handheld ultrasound device 10 including the ultrasound transducer unit may increase. Further, as the number of ultrasound transducers increases, the process of irradiating and receiving ultrasound signals becomes more complex, which requires additional effort and training for maintenance of the ultrasound system. Furthermore, an increase in number of ultrasound transducers leads to an increase in weight and size of the handheld ultrasound device 10, which causes a reduction in portability of the device. Moreover, as the number of ultrasound transducers increases, power consumption during a scan increases. Therefore, the usage time of the handheld ultrasound device 10, which is powered by a battery without a wired connection, is reduced. As a result, it is desirable to set the number of ultrasound transducers in the handheld ultrasound device to 10 or fewer according to an embodiment of the present disclosure.

**A** plurality of ultrasound transducers 310 and 320 included in the ultrasound transducer unit 300 may be arranged in two rows as shown in FIG. 8A. When the ultrasound transducers 310 and 320 in the ultrasound transducer unit 300 are arranged in two or more rows, the ultrasound transducers 310 may be arranged in a zigzag pattern in each row as shown in FIG. 8A. This arrangement can reduce blind spots caused by the spacing between the ultrasound transducers 310 during operation. For example, in areas corresponding to spacings S between adjacent ultrasound transducers 131 in a first row, it may be difficult to accurately receive ultrasound signals with the ultrasound transducers 311 in the first row. However, if ultrasound transducers 312 in a second row are arranged corresponding in position to the spacings S between the ultrasound transducers 311 in the first row, they can receive ultrasound signals in the uncovered areas corresponding to the spacings S.

When the ultrasound transducers of the ultrasound transducer unit 300 are arranged in two or more rows, first and third ultrasound transducers may be arranged in a first row and second ultrasound transducers may be arranged in a second row. In this case, the ultrasound transducers arranged in the second row may be desirably arranged to scan areas corresponding to spacings between the first and third ultrasound transducers. This arrangement allows the second ultrasound transducers to measure blind spots at edges of the first and third ultrasound transducers. Although FIG. 8A and FIG. 8B illustrate an example where the ultrasound transducer unit 300 includes five ultrasound transducers, the number of ultrasound transducers may be adjusted between 3 and 10 depending on the design.

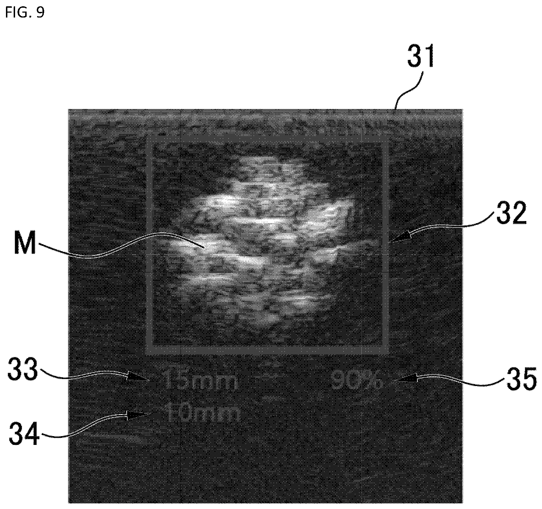

FIG. 9 is another diagram for explaining an interface screen of a mobile device on which ultrasound images generated by the handheld ultrasound device are displayed according to an embodiment of the present disclosure.

More specifically, FIG. 9 illustrates an example of the interface screen displayed on the external device 30 when ultrasound images generated by the handheld ultrasound device 10 is transmitted to the external device 30 through the network 20. Herein, the external device 30 includes a mobile device, such as a smartphone used by the user of the handheld ultrasound device 10, a personal computer, or a medical device.

The external device 30 may use its internal data processing unit to display the received ultrasound images in the form of an image on its display 31. In this case, the display 31 not only presents the processed ultrasound image but also visually distinguishes an area suspected to be a detection target M related to breast cancer by displaying it in a specific shape 32. For example, when a mass suspected of causing breast cancer is found within the ultrasound image, the external device 30 can distinguish its area from the other area by overlaying a bounding box or pixel-level segmentation on the ultrasound image.

To detect a breast mass within the transmitted ultrasound image, the external device 30 may utilize a pre-trained Al neural network. The Al neural network may refer to an Al model that analyzes an input image and outputs a coordinate value of an area corresponding to a breast cancer mass. The Al model can be trained by using image processing algorithms or image classification algorithms, such as convolutional neural networks (CNNs).

Based on the coordinate value of the mass, the external device 30 may display a specific shape or overlay a separate pixel-level image on the image to distinguish the mass from the other area.

Further, the external device 30 may present, on the display 31, an estimated actual size 33 of the detection target, a depth 34 of the detection target within the skin, and a presence probability 35 derived in a process of determining whether the detection target is a tumor related to breast cancer in consideration of a predetermined resolution of the ultrasound image and a predetermined size of the detection target in the image. Herein, the presence probability 35 may refer to the probability that a detected object corresponds to a breast cancer mass, as determined by the Al model.

If the external device 30 functions as an Al cloud server, the user can access the Al cloud server included in the external device 30 by using a separate end device, download data uploaded from the handheld ultrasound device 10 through the Al cloud server, or upload other data to the Al cloud server. The Al cloud server may provide the handheld ultrasound device 10 with commands based on the data uploaded by the user's end device or stored data.

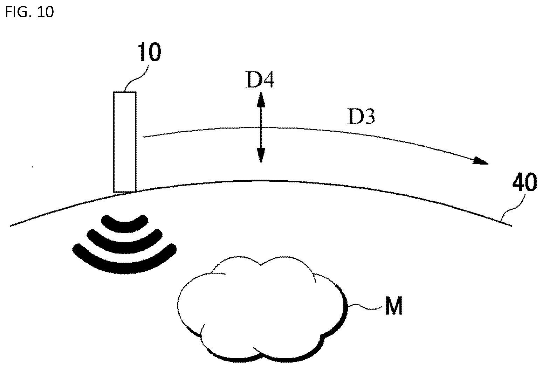

FIG. 10 to FIG. 14 are diagrams for explaining an example where axial movements are performed in the handheld ultrasound device according to an embodiment of the present disclosure.

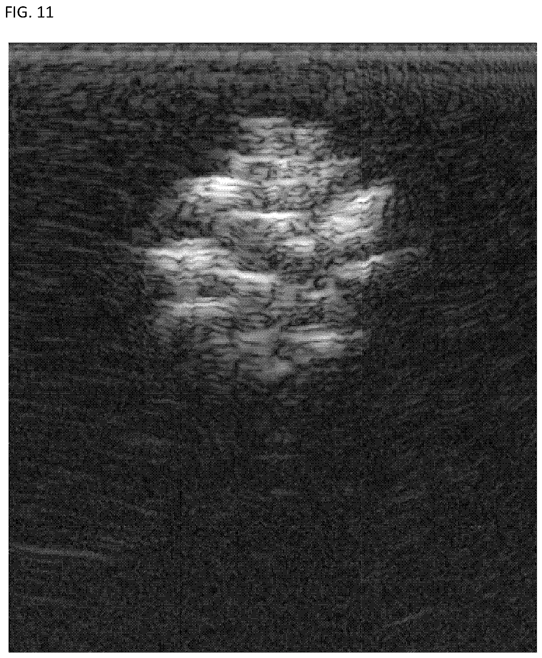

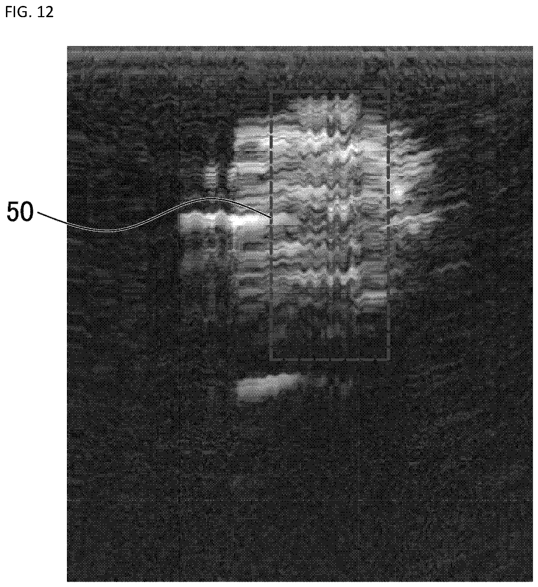

FIG. 10 illustrates a movement direction D3 of the handheld ultrasound device 10 along a breast skin 40 under the control of the user. While scanning to locate the mass M beneath the breast skin 40 by holding the handheld ultrasound device 10, the user may move the handheld ultrasound device 10 in an axial direction D4 due to the user's inattention or variations in breast skin surface level. When the scan is performed on the breast skin without a movement in the axial direction D4, an ultrasound image of the scan target may be generated as shown in FIG. 11. However, when the axial movement D4 occurs during the scan, deformation may occur in a certain area 50 as shown in FIG. 12.

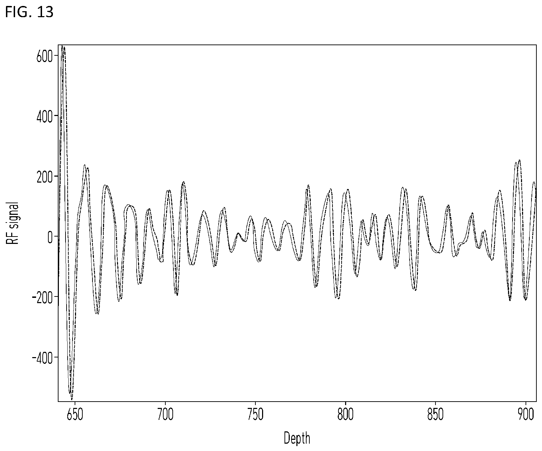

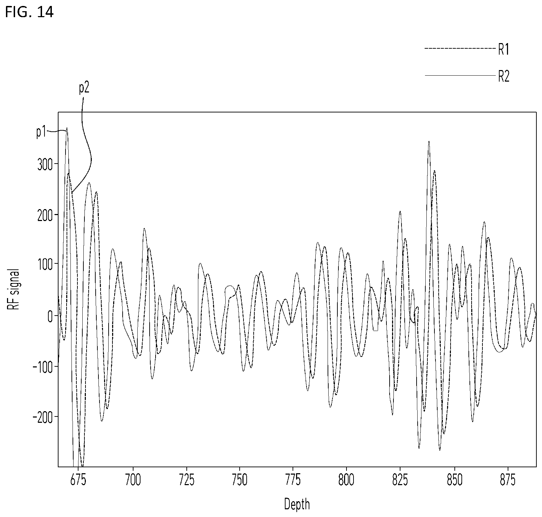

FIG. 13 provides a comparison of adjacent raw signals (RF signals) when the axial movement D4 does not occur during a scan with a handheld breast mass detection device, and FIG. 14 provides a comparison of adjacent raw signals when the axial movement D4 occurs during a scan with the handheld breast mass detection device. It can be seen from FIG. 14 that when the axial movement D4 occurs, a change in movement distance of an ultrasound signal leads to a signal shift along a depth direction.

Referring to FIG. 13 and FIG. 14, each raw signal can be measured in a two-dimensional coordinate system where the X-axis represents a measurement depth for breast mass detection by the handheld ultrasound device 10 and the Y-axis represents an intensity of a signal received by the ultrasound transducer.

When the controller 500 determines that axial movements have occurred while an ultrasound image is generated, it is possible to correct the image as follows to generate a more accurate ultrasound image.

As for a first raw signal R1 and a second raw signal R2 sequentially generated as the handheld ultrasound device 10 moves, the controller 500 measures a first peak value p1 of the first raw signal R1 and derives a first depth value corresponding to the first peak value p1.

Also, the controller 500 measures a second peak value p2 of the second raw signal R2 and derives a second depth value corresponding to the second peak value p2.

Thereafter, the controller 500 calculates a difference between the first and second depth values and generates a correction signal obtained by shifting the second raw signal R2 by the calculated difference. Therefore, it is possible to align the depth values of the peak values in the first raw signal and the correction signal. Then, the controller 500 replaces the second raw signal R2 with the correction signal and thus corrects the image in response to the axial movement.

The above-described method may be divided into additional processes or combined into fewer processes according to the embodiment described above with reference to FIG. 1 to FIG. 14. In addition, some of the processes may be omitted and the sequence of the processes may be changed if necessary.

The above description of the present disclosure is provided for the purpose of illustration, and it would be understood by those skilled in the art that various changes and modifications may be made without changing technical conception and essential features of the present disclosure. Thus, it is clear that the above-described embodiments are illustrative in all aspects and do not limit the present disclosure. For example, each component described to be of a single type can be implemented in a distributed manner. Likewise, components described to be distributed can be implemented in a combined manner.

The scope of the present disclosure is defined by the following claims rather than by the detailed description of the embodiment. It shall be understood that all modifications and embodiments conceived from the meaning and scope of the claims and their equivalents are included in the scope of the present disclosure.

## Claims

1. A handheld ultrasound device configured to detect a breast mass, comprising:
a transducer cover having a contact area to be in contact with a user's breast and located on one end of the handheld ultrasound device;
a contact part whose outer surface is located on at least a portion of the contact area;
an ultrasound transducer unit including N number (N is a natural number of 2 or more) of ultrasound transducers each configured to irradiate an ultrasound signal through the outer surface of the contact part and receive the reflected ultrasound signal;
a controller configured to output a control signal to control the ultrasound transducer unit and generate ultrasound images based on the received ultrasound signals; and
a communication unit configured to transmit the ultrasound images to an external device,
wherein the controller turns on only one ultrasound transducer while turning off the others within one cycle T and generates an ultrasound image based on an ultrasound signal received from the turned-on ultrasound transducer.

2. The handheld ultrasound device of Claim 1,
wherein the controller turns on only a first ultrasound transducer in a first cycle and turns on only a second ultrasound transducer in a second cycle, and on-periods of the first and second ultrasound transducers do not overlap.

3. The handheld ultrasound device of Claim 1,
wherein the cycle T is determined based on a value obtained by dividing a measurement depth of the ultrasound transducer by a propagation speed of ultrasound in the breast.

4. The handheld ultrasound device of Claim 1,
wherein signal phase differences among the N number of ultrasound transducers are determined independently of the number N and a shape of the ultrasound transducers, and a spacing between the ultrasound transducers.

5. The handheld ultrasound device of Claim 1,
wherein the N number of ultrasound transducers of the ultrasound transducer unit sequentially and individually transmits the ultrasound signals to the controller.

6. The handheld ultrasound device of Claim 1,
wherein the controller receives the ultrasound signals sequentially and individually from the N number of ultrasound transducers and generates the ultrasound images sequentially and individually.

7. The handheld ultrasound device of Claim 1,
wherein the control signal is independent of a cross-sectional size of each of the N number of ultrasound transducers and a spacing between the N number of ultrasound transducers.

8. The handheld ultrasound device of Claim 1, further comprising
a movement speed sensing unit configured to sense a movement speed of the contact area in contact with the breast and generate movement speed information.

9. The handheld ultrasound device of Claim 8,
wherein the movement speed sensing unit includes at least one of a speed calculator that calculates a speed of the handheld ultrasound device based on a change in the received ultrasound signal, an acceleration sensor, or an optical sensor.

10. The handheld ultrasound device of Claim 1,
wherein the N is a natural number between 3 and 10, and
the N number of ultrasound transducers are arranged in a zigzag pattern in two rows.

11. The handheld ultrasound device of Claim 10,
wherein first and third ultrasound transducers among the N number of ultrasound transducers are arranged in a first row and a second ultrasound transducer is arranged in a second row and between the first and third ultrasound transducers, and
the second ultrasound transducer measures a blind spot at edges of the first and third ultrasound transducers.

12. The handheld ultrasound device of Claim 1,
wherein the external device is configured to detect a breast mass based on the received ultrasound images and visually distinguish the detected breast mass in a specific shape.

13. The handheld ultrasound device of Claim 1,
wherein the external device utilizes a pre-trained artificial intelligence neural network to detect the breast mass.

14. The handheld ultrasound device of Claim 1,
wherein the external device is configured to display at least one of a size, a depth and a presence probability of the detected breast mass.

15. The handheld ultrasound device of Claim 1,
wherein the external device includes at least one of a smartphone, a personal computer, or a tablet PC connected to the handheld ultrasound device.

16. The handheld ultrasound device of Claim 1,
wherein the external device includes an Al cloud server connected to a plurality of handheld ultrasound devices.

17. The handheld ultrasound device of Claim 1,
wherein the external device includes a medical device connected to a plurality of handheld ultrasound devices.

18. The handheld ultrasound device of Claim 1,
wherein the controller detects an axial movement along a vertical direction while the contact area moves in contact with the breast in a horizontal direction, and generates the ultrasound images by correcting the received ultrasound signals based on the detected axial movement.

19. The handheld ultrasound device of Claim 1,
wherein the N is a natural number between 2 and 10.
